# EUROPEAN PATENT SPECIFICATION

(11) **EP 3 164 086 B1**
(45) Date of publication and mention of the grant of the patent: **08.04.2020**
(21) Application number: 15739407.3
(22) Date of filing: 30.06.2015
(51) Int. Cl.: A61B 17/128, A61M 25/00

(54) **OVERLAPPED BRAID TERMINATION**
ÜBERLAPPENDES GEFLOCHTENES ENDSTÜCK
EXTRÉMITÉ DE TRESSE SE CHEVAUCHANT

(30) Priority: 01.07.2014 US 201462019582 P
(43) Date of publication of application: 10.05.2017
(73) Proprietor: Boston Scientific Scimed, Inc., Maple Grove, MN 55311-1566 (US)
(72) Inventor: LILBURN, Steven H., Princeton, Massachusetts 01541 (US); WILCOX, Kevin J., Brighton, Massachusetts 02135 (US)
(74) Representative: Vossius & Partner Patentanwälte Rechtsanwälte mbB
(86) International application number: PCT/US2015/038558
(87) International publication number: WO 2016/004041

(56) References cited:
- WO-A1-98/56447
- JP-A- 2007 089 847
- US-A1- 2004 092 962
- US-A1- 2004 176 740
- US-A1- 2005 192 581
- US-A1- 2008 262 472
- US-A1- 2009 227 932
- US-B1- 7 905 877

## Description

### Background

Endoscopic medical devices such as, for example, hemostatic clipping devices, comprise a clip coupled via a flexible shaft member to a handle member which remains outside of patient's body while the flexible shaft and the clip are inserted to a target site (e.g., through an endoscope or other insertion device passed into a body lumen via a natural body orifice). The flexible shaft member may be formed, for example, as a coil of wire or other flexible structure to facilitate insertion of the clip into the body via along tortuous paths. When the clip has reached a target site, physicians may wish to rotate the clip to a desired orientation by rotating the handle or the proximal end of the flexible shaft. These flexible members may, at times, inefficiently transmit torque applied at the proximal end of the flexible member to the clip at the distal end making it difficult for the physician to orient the clip as desired. To enhance the transmission of torque along the flexible member some devices have included a braided member over an outer surface of the coil of the flexible member.

In US 7,905,877 B1 an intravascular catheter is disclosed, which comprises a polymeric inner tube, a reinforcing jacket which is spirally wound over the inner tube and which becomes progressively softer from a proximal end to a distal end, and a polymeric outer sheath extruded over the inner jacket. Methods for making the catheter and for annealing ends of the wound wire are also disclosed.

### Summary

The invention is directed to a medical device, comprising a flexible shaft extending along a longitudinal axis from a distal end to a proximal end, the shaft including a first reduced cross-sectional area portion extending along a distal portion of a length thereof and a braid applied overlappingly over a portion of the reduced cross-sectional area portion of the shaft, a first layer of the braid applied proximally over the shaft from a braid distal end to a first braid end point distal of a proximal end of the first reduced cross-sectional area portion, a second layer of the braid applied distally over the first layer from the first braid end point to a second end point proximal of the braid distal end, and a third layer applied proximally over the second layer proximally beyond the proximal end of the reduced cross-sectional area to a third braid end point along the shaft.

### Brief Description of the Drawings

Fig. 1 shows a perspective view of a device according to an exemplary embodiment of the present disclosure;
Fig. 2 shows a side view of the device of Fig. 1, in a first state;
Fig. 3 shows a side view of the device of Fig. 1, in a second state;
Fig. 4 shows a side view of the device of Fig. 1, in a third state;
Fig. 5 shows a braiding system according to an exemplary embodiment of the present disclosure;

### Detailed Description

The present disclosure may be further understood with reference to the following description and the appended drawings, wherein like elements are referred to with the same reference numerals. The present disclosure is directed to a flexible, medical device including a braided shaft. In particular, exemplary embodiments of the present disclosure describe an endoscopic medical device comprising a flexible shaft including a braid extending along a portion of a length thereof so that torque may be transmitted along the length of the shaft. For example, a hemostatic clipping device may include a clip coupled to a handle member via a flexible shaft including a braid extending over a portion of a length thereof. Braids formed of non-annealed wire or filaments have been difficult to terminate cleanly as the filaments of the braid often spring away from a surface of the shaft upon cutting. A system and method according to an exemplary embodiment of the present disclosure includes overlapping layers of braids in a particular configuration over the shaft to provide a secure and atraumatic termination the braid at a distal end of the shaft. It will be understood by those of skill in the art that although the exemplary embodiments specifically describe a hemostatic clipping device, the braid termination of the present disclosure may be used in any medical device including a braid covering such as, for example, a stent. It should be noted that the terms "proximal" and "distal" as used herein, are intended to refer to a direction toward (proximal) and away from (distal) a user of the device.

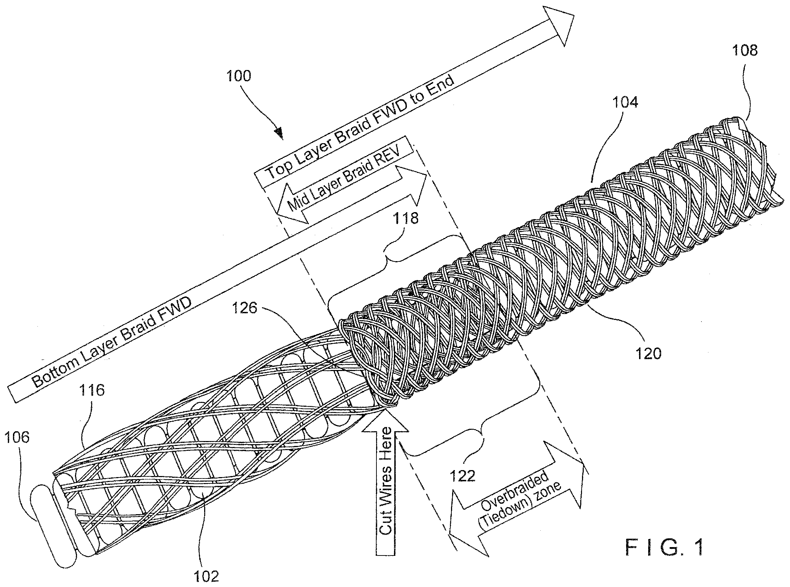

As shown in Figs. 1 - 4, a device 100 according to an exemplary embodiment of the present disclosure comprises a shaft 102 including a braid 104 extending over a portion of a length thereof. The braid 104 is formed of a plurality of wires or other flexible filaments that braided over an exterior surface of the shaft 102. The shaft 102 may, for example, extend between a clipping portion (not shown) of the device 100 at a distal end 106 thereof and a handle portion (not shown) of the device 100 at a proximal end 108 thereof. The shaft 102 may be in the form of a coil of flexible wire or any other suitable structure extending along a longitudinal axis from the distal end 106 to the proximal end 108. The braid 104 may include overlapping layers including a first layer 116 extending from the distal end 106 toward the proximal end 108 for a first distance, a second layer 118 extending distally over a portion of the first layer 116 and a third layer extending proximally over the second layer1 18 toward the proximal end 108 of the shaft 102. The overlapping layers 116, 118, 120 of the braid 104, as described in further detail below, are specifically designed to provide a secure and atraumatic braid termination of the braid 104 while imparting the desired torque transmission along the shaft 102.

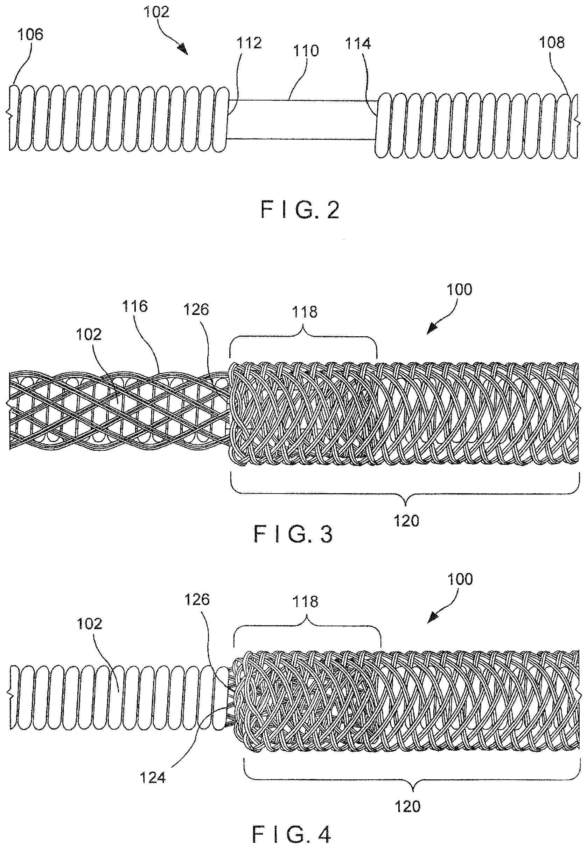

As shown in Fig. 2, the shaft 102 includes a termination portion 110 with a reduced cross-section (e.g., reduced diameter). As would be understood by those skilled in the art, the diameter of this portion of the shaft 102 is preferably reduced by an amount equal to a total thickness of the braid 104 so that, when covered by the braid 104 an outer profile of the shaft 102 is substantially smooth. The termination portion 110 extends along a portion of a length of the shaft 102 from a distal end 112 toward a proximal end 114 and corresponds in length to an overlapping portion 122 of the braid 104, selected to achieve the desired braid termination. Distal and proximal ends 112, 114 of the termination portion 110 are separated from the distal and proximal ends 106, 108 of the shaft 102, respectively.

As shown in Figs. 1 and 3, the first layer 116 of the braid 104 is applied over the shaft 102 from the distal end 106 toward the proximal end 108 to a position along the shaft 102 distal of the proximal end 114 of the termination portion 110. The first layer 116 is applied over the shaft 102 for a short distance (e.g., 1.5 mm to 4.0 mm) at a small braid angle. The first layer 116 would preferably have a zero braid angle (i.e., extending parallel to a longitudinal axis of the shaft 102) to provide the least amount of bulk to the first layer 116 and to provide the shortest trimmed length, as will be described in greater detail below. It will be understood by those of skill in the art, however, that it may be difficult to get a true zero angle braid to grip the shaft 102 as a true zero angle braid would not provide any wrapping about the shaft 102. Thus, although the braid angle of the first layer 116 should be as small as possible, the braid angle of the first layer 116 may range from 0 degrees up to 90 degrees and more particularly, between 50 and 60 degrees. A braid angle may be defined as an angle between strands of the braid 104. Thus, while strands of the first layer 116 of the braid 104 may be angled relative to one another at an angle of up to 90 degrees, it will be understood by those of skill in the art that a 90 degree braid angle would correspond to a 45 degree angle relative to the longitudinal axis of the shaft 102. The braid angle of the first layer 116 may also vary along a length of the shaft 102. For example, the braid angle of the first layer 116 may be increased along the termination portion 110 of the shaft 102 to provide a better grip thereto. In particular, the braid angle of the first layer 116 may be increased along a short distance (e.g., 2 mm) of a portion of the termination portion 110 immediately distal the proximal end 114 thereof to increase a grip of the first layer 116 as a braid direction is reversed to apply the second layer 118 thereto. It will be understood by those of skill in the art that this increased braid angle, however, will result in added bulk to the shaft 102.

A direction of the braid 104 is then reversed so that the second layer 118 is then applied distally over a portion of the first layer 116 to a position along the shaft 102 proximal of the distal end 112 of the termination portion 110. A braid angle of the second layer 118 may be selected to best lock the first layer 116 to the shaft 102. The braid angle of the second layer 118 should also be selected to be as small as practicable to prevent adding any unnecessary bulk to the shaft 102. The braid angle of the second layer 118 may be between 90 degrees (i.e., 45 degrees relative to the longitudinal axis of the shaft 102) and 180 degrees (i.e., 90 degrees relative to the longitudinal axis of the shaft 102) and, more particularly, between 140 and 150 degrees. The direction of the braid 104 is once again reversed so that the third layer 120 of the braid 104 is applied proximally over the second layer 118 toward the proximal end 108 of the shaft 102 to a desired point therealong. A braid angle of the third layer 120 may be selected to provide the desired level of securement of the first and second layers 116, 118 while also providing the desired level of torsional transmission to the shaft 102. The braid angle of the third layer 120 may also range between 90 degrees and 180 degrees and, more particularly, between 140 and 150 degrees. Braid angles of the first, second and third layers 116, 118, 120, and/or a combination thereof may also be selected to increase a strength of the termination portion 110, which has a reduced cross-sectional area. A braid angle particularly suited for increasing a strength of the termination portion may be less than 135 degrees. Thus, the overlapping portion 122 of the braid 104 (i.e., the portion of the braid 104 in which all of the layers 116, 118, 120 overlap one another) is entirely between the distal and proximal ends 112, 114 of the termination portion 110. It will be understood by those of skill in the art that the first, second and third layers 116, 118, 120 form one continuous braid 104. It will also be understood by those of skill in the art that the overlapping portion 122 is applied over the reduced cross-section termination portion 110 of the shaft 102 so that an outer-most cross-sectional area (e.g., outermost diameter) of the overlapped portion 122 preferably does not extend beyond a cross-sectional area of a portion of the braid 104 extending proximally thereof along a proximal portion of the shaft 102.

Once the three layers 116, 118, 120 have been applied over the shaft 102, as desired, a portion of the braid 104 extending distally from the overlapped portion 122 (i.e., portion of the first layer 116 extending distally from the end of the overlapping portion 122) is cut to form a clean braid termination 124 at a distal end thereof, as shown in Fig. 4. The braid 104 is cut immediately distal to a distal edge 126 of the second layer 118 such that the distal braid termination 124 is formed within the termination portion 110 of the shaft 102 - i.e., proximally of the distal end 112 of the termination portion 110. It will be understood by those of skill in the art that a smaller braid angle of the first layer 116 will result in a smaller length of cut braid extending distally from the overlapped portion 122. In particular, where the braid angle of the first layer 116 is 0 degrees, the length of the braid extending distally from the overlapped portion is equal to a distance between the distal braid termination 124 and a distal-most edge of the overlapped portion 122 (e.g., cut distance). Where the braid angle of the first layer 116 is 90 degrees (i.e., 45 degrees relative to the longitudinal axis of the shaft 102), however, the length of the cut braid extending distally from the overlapped portion 122 is equal to the cut distance/sin 45°, thereby resulting in a longer length of cut braid extending therefrom. Thus, as discussed above, the braid angle of the first layer 116 may be selected to provide the least amount of bulk and to provide the shortest trimmed length. It will be understood by those of skill in the art that the trimmed length of the first layer 116 is short enough such that the distal termination end 124 cannot extend beyond an outer diameter of the overlapped portion 122 and is formed within the termination portion 110. Thus, it will be understood by those of skill in the art that the distal termination portion 124 is both secure (e.g., mechanically attached to the shaft 102 via the second and third layers 118, 120) and atraumatic (e.g., within and secured to the termination portion 110 to prevent edges of the distal braid termination 124 from catching on any surface through which the shaft 102 is inserted).

It will be understood by those of skill in the art that the proximal end 108 of the shaft 102, and thereby a proximal end of the braid 104, is often encapsulated within the handle portion of the device 100 such that a clean proximal braid termination is not required. However, it will also be understood by those of skill in the art that, if desired, the proximal end of the braid may be terminated in a manner substantially similar to the distal braid termination 124 described above. In particular, the third layer 120 of the braid 104 extends proximally along the length of the shaft 102 to a desired proximal point along the shaft 102. Upon reaching the desired proximal point, the braid 104 reverses directions so that a fourth layer extends distally over a proximal portion of the third layer 104 for a desired distance. The braid 104 may then reverse directions again such that fifth layer extends proximally over the fourth layer toward the proximal end 108 of the shaft 102. The braid 104 may then be cut at a point proximally of the proximal edge of the fourth layer such that proximal edges of the braid 104 may be folded over the proximal edge of the fourth layer and inserted between an exterior surface of the shaft 102 and an interior surface of the third layer 120 to create a clean proximal edge of the braid 104. Similarly to the overlapping layers of braid 104 at the distal end thereof, the overlapping layers at the proximal end of the braid 104 may also extend over a termination portion of the shaft 102 having a smaller cross-section than a remaining portion of the shaft 102 such that the overlapping layers at the proximal end of the braid 104 do not result in a larger cross-sectional area than a remaining portion of the braid 104. Alternatively, the proximal end of the shaft 102 may not include a termination portion such that the overlapping layers at the proximal end may result in a larger cross-sectional area thereover. This larger cross-sectional area may provide an axial anchor for attaching a handle and/or provide a rotational connection.

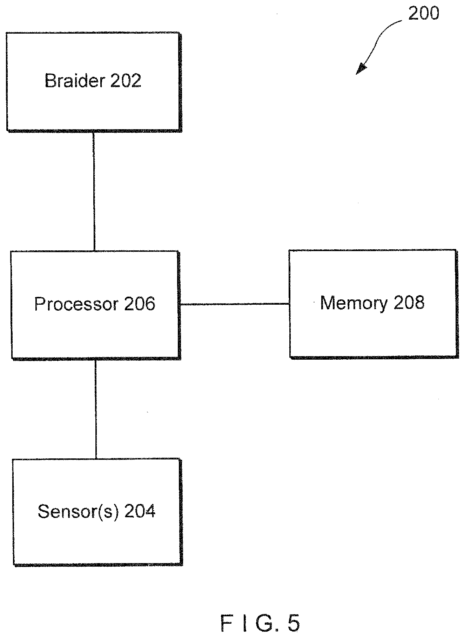

As shown in Fig. 5, a braiding system 200 for applying the braid 104 over the shaft 102 of the device 100 according to an exemplary embodiment of the present disclosure comprises a braider 202 for braiding wires or other filaments of the braid 104 over a portion of the shaft 102, one or more sensors 204 for detecting the distal end 106 of the shaft 102 along with the distal and proximal ends 112, 114 of the termination portion 110 and a processor 206 for determining a desired length and position of each of the layers 116, 118, 120. The system 200 also comprises a memory 208 for storing a set of instructions executable by the processor 206 for applying the braid 104 over the shaft 102. Information such as, for example, detected and/or calculated points along the shaft 102 (e.g., distal and proximal ends of the termination portion 110, points on the shaft 102 where layers 116, 118, 120 should begin and end) and braid angles of the various layers 116, 118, 120 may also be stored on the memory 208. The braiding system 200 may further comprise a cutter for cutting the braid 104 to form a braid termination 124.

The braider 202 receives the distal end 106 of the shaft 102 to apply the braid 104 from the distal end 106 toward the proximal end 108 of the shaft 102. It will be understood by those of skill in the art that the braider 202 may include a motor for moving the shaft 102 relative to the braider 202, braiding arms for applying the braid 104 of wires or other flexible filaments over the shaft 102 moving longitudinally therewithin and any other features known in the art for application of a braid over a shaft. In particular, the braider 202 applies the braid 104 in a proximal direction as the shaft 102 is moved distally relative thereto. The sensor(s) 204 detects the distal end 106 of the shaft 102 as the shaft 102 approaches a braiding area of the braider 202. It will be understood by those of skill in the art that the braider 202 may either be running or waiting for a shaft 102 to be loaded. Using the detected distal end 106 of the shaft 102 and/or a desired braid angle of the braid 104, the processor 206 may determine a beginning point along the shaft 102 at which to begin braiding. A relative position of the shaft 102 and the braider 104 may be automatically determined by the processor 206 based on input parameters or may be overriden with values empirically developed. The braider 202 may initially apply a short section (e.g., approximately 3mm) of braid 104 from the beginning point toward the proximal end 108 at a braid angle high enough to secure the braid 104 to the shaft 102. Once the short section of braid has been applied, the braider 202 applies the first layer 116 of the braid 104 proximally along the shaft 102. A wire clamp may be applied over the braid 104 on the shaft 102 at the start/end of any section of braid 104 to enable sharp changes in braid angle between sections. In another example, the braider 202 may also be stopped for sections, as desired, to enable a braid angle of zero between sections. For example, the braider 202 may be stopped between the short section of high braid angle and the lower braid angle of the first layer 116.

Before or during the application of the first layer 116, the sensor(s) 204 detect features of the shaft 102 indicating the termination portion 110. For example, the sensors 204 may detect cross-sectional changes in the shaft 102 to identify distal and proximal ends 112, 114 of the termination portion 110. The distal and proximal ends 112, 114 of the termination portion 110 may be used to calculate a length and position of the termination portion 110 along the shaft 102. Using this information, the processor 206 determines a desired end point of the first layer 116 and/or a length of the first layer 116 to be applied so that the braider 202 may apply the first layer 116 of the braid 104 proximally along the shaft 102 over the termination portion 110 to a point distal of the proximal end 114 of the termination portion 110. Features of the termination portion 110 may also be used to calculate end points of the second layer 118 and/or a length of the second layer 118 to be applied distally over the first layer 116. It will be understood by those of skill in the art that there may be more than one sensor 204 for detecting the various features of the shaft 102 such as, for example, the distal end 106 of the shaft 102 and the distal and proximal ends 112, 114 of the termination portion 110.

As described above, the first layer 116 is applied at a low braid angle to facilitate cutting of the braid 104 at the braid termination point 124. The braider 202 applies the first layer 116 to the calculated end point thereof and may additionally apply a short section of braid at a higher braid angle proximally of the calculated end point to secure the proximal end of the first layer 116 to the shaft 102 and prevent the braid 104 from sliding when the braid direction is reversed for application of the second layer 118 of the braid 104. It will be understood by those of skill in the art that the braid applied at the higher braid angle at the proximal end of the first layer 116 should not extend proximally beyond the proximal end 114 of the termination portion 110. The braider 202 then reverses a direction of the shaft 102 so that the second layer 118 of the braid 104 is applied distally over the first layer 116 to the calculated distal end thereof (e.g., within the bounds of the termination portion 110). The shaft 102 is moved proximally so that the second layer 118 may be applied distally over the first later 116. The braider 202 then reverse the direction of the shaft 102 so that the shaft 102 is once again moved distally with respect to the braider 102 to apply the third layer 120 of the braid 104 thereover, in a proximal direction. As described above, the third layer 120 is applied proximally over the second layer 118 and a portion of the first layer 116 at a braid angle sufficiently high enough to secure the underlying layers 116, 118 of braid 104 to the shaft 102. The third layer 120 extends proximally along the shaft 102 to a desired proximal point thereof. This desired proximal point may be stored in the memory 208 as an input parameter based on a desired use of the device 100. Once all of the layers 116, 118, 120 have been applied over the shaft 102, as desired, the portion of braid extending distally beyond the distal edge 126 of the second layer 118 may be cut to form the braid termination 124.

As described above, loose ends of the braid 104 at the proximal end of the third layer 120 may be captured and encapsulated within a handle portion of the device 100 so that an overlapping braid configuration is not required. If, however, a braid termination similar to the braid termination 124 is desired at the proximal end of the braid 104, the overlapped braid configuration may be similarly applied over a termination portion along a proximal portion of the shaft 102.

It will be apparent to those skilled in the art that various modifications may be made in the present disclosure, without departing from the scope of the disclosure. Thus, it is intended that the present disclosure cover modifications and variations of this disclosure provided that they come within the scope of the appended claims and their equivalents.

## Claims

1. A medical device (100), comprising:
a flexible shaft (102) extending along a longitudinal axis from a distal end (106) to a proximal end (108), the flexible shaft (102) including a first reduced cross-sectional area portion extending along a distal portion of a length thereof; and
a braid (104) applied overlappingly over a portion of the first reduced cross-sectional area portion of the flexible shaft (102), a first layer (116) of the braid (104) applied proximally over the flexible shaft (102) from a braid distal end to a first braid end point distal of a proximal end of the first reduced cross-sectional area portion, a second layer (118) of the braid (104) applied distally over the first layer (116) from the first braid end point to a second end point proximal of the braid distal end, and a third layer (120) applied proximally over the second layer (118) proximally beyond the proximal end of the first reduced cross-sectional area to a third braid end point along the shaft (102).

2. The medical device (100) of claim 1, wherein a distal braid termination is formed by cutting away a portion of the first layer (116) extending distally beyond a distal edge of the second layer (118) of the braid.

3. The medical device (100) of claim 2, wherein a braid angle of the first layer (116) is less than 90 degrees and is selected to be so that the portions of the first layer (116) extending distally beyond the distal edge of the first layer (116) are easily cuttable from a remaining portion of the braid.

4. The medical device (100) of any of claims 1 to 3, wherein a braid angle of the second layer (118) is greater than 90 degrees to sufficiently secure the first layer (116) to the shaft (102).

5. The medical device (100) of any of claims 1 to 4, wherein a braid angle of the third layer (120) is greater than 90 degrees to be sufficiently high to secure the first (116) and second layers (118) of the braid to the flexible shaft (102).

6. The medical device (100) of any of claims 1 to 5, wherein a diameter of the first reduced cross-sectional area portion of the flexible shaft (102) is reduced relative to other portions of the flexible shaft (102) by an amount equal to a total thickness of the braid so that an outer profile of the flexible shaft (102) including the braid is smooth.

7. The medical device (100) of any of claims 1 to 6 , wherein the flexible shaft (102) further includes a second reduced cross-section area along a proximal portion thereof.

8. The medical device (100) of claim 7, wherein the third braid end point is distal of a proximal end of the second reduced cross-sectional area and the braid further includes a fourth layer extending distally over a portion of the third layer (120) to a fourth braid end point proximal of a distal end of the second reduced cross-sectional area and a fifth layer extending distally over the fourth layer from the fourth braid end point to a proximal end that is folded over a proximal edge of the fourth layer and inserted between the braid and the flexible shaft (102).

## Patentansprüche

1. Medizinische Vorrichtung (100), aufweisend:
einen biegsamen Schaft (102), der sich entlang einer Längsachse von einem distalen Ende (106) zu einem proximalen Ende (108) erstreckt, wobei der biegsame Schaft (102) einen ersten Abschnitt mit verringerter Querschnittsfläche entlang eines distalen Abschnitts seiner Länge umfasst; und
ein Geflecht (104), das überlappend auf einen Teil des ersten Abschnitts mit verringerter Querschnittsfläche des biegsamen Schafts (102) aufgebracht ist, wobei eine erste Schicht (116) des Geflechts (104) proximal von einem distalen Geflechtende zu einem ersten Geflechtendpunkt, der sich distal zu einem proximalen Ende des ersten Abschnitts mit verringerter Querschnittsfläche befindet, auf den biegsamen Schaft (102) aufgebracht ist, eine zweite Schicht (118) des Geflechts (104) distal vom ersten Geflechtendpunkt zu einem zweiten Endpunkt, der sich proximal zum distalen Geflechtende befindet, auf die erste Schicht (116) aufgebracht ist und eine dritte Schicht (120) proximal über das proximale Ende des ersten Abschnitts mit verringerter Querschnittsfläche hinaus bis zu einem dritten Geflechtendpunkt entlang des Schafts (102) auf die zweite Schicht (118) aufgebracht ist.

2. Medizinische Vorrichtung (100) nach Anspruch 1, wobei ein distaler Geflechtabschluss durch Wegschneiden eines Teils der ersten Schicht (116), der sich distal über einen distalen Rand der zweiten Schicht (118) des Geflechts erstreckt, gebildet wird.

3. Medizinische Vorrichtung (100) nach Anspruch 2, wobei ein Geflechtwinkel der ersten Schicht (116) kleiner als 90 Grad und so gewählt ist, dass die Teile der ersten Schicht (116), die sich distal über den distalen Rand der ersten Schicht (116) hinaus erstrecken, leicht von einem verbleibenden Teil des Geflechts abgeschnitten werden können.

4. Medizinische Vorrichtung (100) nach einem der Ansprüche 1 bis 3, wobei ein Geflechtwinkel der zweiten Schicht (118) größer als 90 Grad ist, um die erste Schicht (116) ausreichend sicher am Schaft (102) zu befestigen.

5. Medizinische Vorrichtung (100) nach einem der Ansprüche 1 bis 4, wobei ein Geflechtwinkel der dritten Schicht (120) größer als 90 Grad ist, damit er groß genug ist, um die erste Schicht (116) und die zweite Schicht (118) des Geflechts am Schaft (102) zu befestigen.

6. Medizinische Vorrichtung (100) nach einem der Ansprüche 1 bis 5, wobei ein Durchmesser des ersten Abschnitts mit verringerter Querschnittsfläche des biegsamen Schafts (102) in Bezug auf andere Abschnitte des biegsamen Schafts (102) um einen Betrag reduziert ist, der einer Gesamtdicke des Geflechts entspricht, so dass ein Außenprofil des biegsamen Schafts (102) einschließlich des Geflechts glatt ist.

7. Medizinische Vorrichtung (100) nach einem der Ansprüche 1 bis 6, wobei der biegsame Schaft (102) entlang seines proximalen Abschnitts ferner eine zweite verringerte Querschnittsfläche umfasst.

8. Medizinische Vorrichtung (100) nach Anspruch 7, wobei sich der dritte Geflechtendpunkt distal zu einem proximalen Ende der zweiten verringerten Querschnittsfläche befindet und das Geflecht ferner eine vierte Schicht, die sich distal über einen Abschnitt der dritten Schicht (120) bis zu einem vierten Geflechtendpunkt proximal von einem distalen Ende der zweiten verringerten Querschnittsfläche erstreckt, und eine fünfte Schicht umfasst, die sich distal über die vierte Schicht vom vierten Geflechtendpunkt zu einem proximalen Ende erstreckt, das über einen proximalen Rand der vierten Schicht gefaltet und zwischen dem Geflecht und dem biegsamen Schaft (102) eingefügt ist.

## Revendications

1. Dispositif médical (100) comprenant :
une tige flexible (102) s'étendant le long d'un axe longitudinal depuis une extrémité distale (106) jusqu'à une extrémité proximale (108), la tige flexible (102) incluant une première partie à aire en coupe transversale réduite s'étendant le long d'une partie distale d'une longueur de celle-ci ; et
une tresse (104) appliquée de manière chevauchante au-dessus d'une partie de la première partie à aire en coupe transversale réduite de la tige flexible (102), une première couche (116) de la tresse (104) appliquée de manière proximale au-dessus de la tige flexible (102) depuis une extrémité distale de la tresse jusqu'à un premier point d'extrémité de la tresse distale d'une extrémité proximale de la première partie à aire en coupe transversale réduite, une seconde couche (118) de la tresse (104) appliquée de manière distale au-dessus de la première couche (116) depuis le premier point d'extrémité de la tresse jusqu'à un second point d'extrémité proximal de l'extrémité distale de la tresse, et une troisième couche (120) appliquée de manière proximale au-dessus de la seconde couche (118) de manière proximale au-delà de l'extrémité proximale de la première aire en coupe transversale réduite jusqu'à un troisième point d'extrémité de la tresse le long de la tige (102).

2. Dispositif médical (100) selon la revendication 1, où une terminaison de la tresse distale est formée par retrait par coupure d'une partie de la première couche (116) s'étendant de manière distale au-delà d'un bord distal de la seconde couche (118) de la tresse.

3. Dispositif médical (100) selon la revendication 2, où un angle de tresse de la première couche (116) est inférieur à 90 degrés et est choisi pour être tel que les parties de la première couche (116) s'étendant de manière distale au-delà du bord distal de la première couche (116) puissent être coupées facilement d'une partie restante de la tresse.

4. Dispositif médical (100) selon l'une quelconque des revendications 1 à 3, où un angle de tresse de la seconde couche (118) est supérieur à 90 degrés pour fixer suffisamment la première couche (116) à la tige (102).

5. Dispositif médical (100) selon l'une quelconque des revendications 1 à 4, où un angle de tresse de la troisième couche (120) est supérieur à 90 degrés pour être suffisamment élevé pour fixer la première couche (116) et la seconde couche (118) de la tresse à la tige flexible (102).

6. Dispositif médical (100) selon l'une quelconque des revendications 1 à 5, où un diamètre de la première partie à aire en coupe transversale réduite de la tige flexible (102) est réduit par rapport à d'autres parties de la tige flexible (102) d'une quantité égale à une épaisseur totale de la tresse de sorte qu'un profil externe de la tige flexible (102) incluant la tresse est uniforme.

7. Dispositif médical (100) selon l'une quelconque des revendications 1 à 6, où la tige flexible (102) inclut en outre une seconde aire en coupe transversale réduite le long d'une partie proximale de celle-ci.

8. Dispositif médical (100) selon la revendication 7, où le troisième point d'extrémité de la tresse est distal d'une extrémité proximale de la seconde aire en coupe transversale réduite et la tresse inclut en outre une quatrième couche s'étendant de manière distale au-dessus d'une partie de la troisième couche (120) jusqu'à un quatrième point d'extrémité de la tresse proximale d'une extrémité distale de la seconde aire en coupe transversale réduite et une cinquième couche s'étendant de manière distale au-dessus de la quatrième couche depuis le quatrième point d'extrémité de la tresse jusqu'à une extrémité proximale qui est pliée au-dessus d'un bord proximal de la quatrième couche et insérée entre la tresse et la tige flexible (102).
